# FASCICULE DE BREVET EUROPEEN

(11) **EP 2 401 018 B1**
(45) Date de publication et mention de la délivrance du brevet: **25.06.2014**
(21) Numéro de dépôt: 10709909.5
(22) Date de dépôt: 25.02.2010
(51) Int. Cl.: A61M 25/00, A61M 5/32

(54) **VECTEUR D'INJECTION D'UN PRODUIT DANS LE CORPS HUMAIN**
TRÄGER ZUR INJEKTION EINES PRODUKTS IN DEN MENSCHLICHEN KÖRPER
CARRIER FOR INJECTING A PRODUCT INTO THE HUMAN BODY

(30) Priorité: 25.02.2009 FR 0951194
(43) Date de publication de la demande: 04.01.2012
(73) Titulaire: Persat, Jean-Charles, 1239 Collex-bossy (CH)
(72) Inventeur: Persat, Jean-Charles, 1239 Collex-bossy (CH)
(74) Mandataire: Thibault, Jean-Marc
(86) Numéro de dépôt international: PCT/FR2010/050326
(87) Numéro de publication internationale: WO 2010/097551

(56) Documents cités:
- WO-A1-2008/155145
- WO-A2-01/97743
- DE-U1- 9 400 470
- US-A- 2 634 726
- US-A- 4 413 993
- US-A- 5 919 170
- US-A- 6 074 371
- US-A1- 2005 192 560

## Description

La présente invention concerne le domaine technique général des vecteurs d'injection d'un produit dans le corps humain et elle vise plus précisément les vecteurs d'injection utilisés dans le domaine de la chirurgie esthétique non invasive par voie transcutanée.

Cette technique de chirurgie esthétique consiste à réparer des défauts esthétiques cutanés, du regard, de la silhouette, du contour du visage et du corps, en général. La technique de traitement consiste à réduire les défauts esthétiques par comblement des différentes couches cutanées, sous-cutanées, musculaires et péri-osseuses soit par la greffe autologue des propres adipocytes du patient (cellules graisseuses prélevées sur les sites anatomiques les plus riches en graisse), soit par l'injection et l'infiltration des espaces tissulaires d'un produit de comblement de synthèse tel que l'acide hyaluronique qui se présente sous la forme d'un gel hydrophile avec différents niveaux de viscosité.

Dans l'état de la technique, il est connu pour assurer l'injection d'un produit de comblement, d'utiliser des canules droites dites canules de Coleman afin de procéder aux injections aux endroits désirés. L'utilisation de telles canules entraîne un certain nombre d'inconvénients comme l'apparition de douleurs et le déchirement des muscles peauciers.

Pour tenter d'apporter une solution à l'utilisation de ces canules notamment dans le domaine de la reconstruction du visage, le brevet FR 2 808 208 a proposé un set de canules pour injection mettant en oeuvre trois canules de configurations géométriques distinctes adaptées aux différentes parties du visage. De manière générale, chaque canule comporte une aiguille tubulaire semi-rigide présentant une extrémité distale hémisphérique fermée et une extrémité proximale de raccordement à un dispositif d'injection. Cette aiguille comporte par ailleurs une ouverture latérale de sortie aménagée à proximité de l'extrémité distale. Même si ces canules possèdent une extrémité mousse atraumatique, il est constaté des traumatismes tissulaires multiples avec un risque multiple d'infections, de microlésions, de micro-hématomes cutanés pouvant laisser des séquelles disgracieuses et définitives (sous forme de piqueté hémorragique avec des marques micro-mématomes). Par ailleurs, l'injection avec une telle canule conduit à l'injection irrégulière du produit qui se répartit sous une forme de chapelet. Ce résultat est disgracieux et demande un massage local après injection pour tenter d'en éliminer l'impact. Il est à noter également une surconsommation du produit injecté du fait de la formation de chapelet avec des grosses gouttes de produit. Cette canule présente par ailleurs une fragilité rendant délicate son utilisation.

Dans l'état de la technique, il est également connu des aiguilles hypodermiques conçues pour assurer une injection en profondeur. Par exemple, le brevet US 2 634 726 décrit une aiguille hypodermique ayant une extrémité biseautée et une ouverture latérale de sortie incurvée. De même, le document DE 94 00 470 U1 décrit également une aiguille hypodermique comportant une extrémité biseautée et une diminution de section en regard de l'ouverture latérale de sortie. Le brevet US 4 413 993 décrit une aiguille pour injection intraveineuse présentant une extrémité biseautée. Toutes ces aiguilles ne sont pas adaptées pour le domaine de la chirurgie esthétique non invasive par voie transcutanée.

Dans l'état de la technique, il est également connu des cathéters urinaires notamment par les documents US 2005/0192560, US 5 919 170 et WO 2008/155145. Ces cathéters comportent un tube dont une extrémité distale est arrondie et en amont duquel est aménagée une ouverture de passage. De tels cathéters ne sont pas adaptés pour servir de vecteur atraumatique d'injection transcutanée d'un produit selon un écoulement régulier.

L'objet de l'invention vise donc à remédier aux inconvénients énoncés ci-dessus en proposant un vecteur d'injection pour la microplastie ou la lipo-reconstruction, de conception robuste et sûre, assurant un écoulement régulier du produit injecté et dont l'utilisation est atraumatique et ne conduit pas à l'apparition de douleurs et d'hématomes chez les patients.

Pour atteindre un tel objectif, l'objet de l'invention concerne un vecteur d'injection d'un produit de remplissage notamment pour la microplastie ou la lipo-reconstruction, comportant une aiguille tubulaire semi-rigide d'axe longitudinal présentant une extrémité distale hémisphérique fermée et une extrémité proximale de raccordement à un dispositif d'injection, cette aiguille présentant une face interne tubulaire délimitant une section droite transversale interne et une face externe tubulaire délimitant une section droite transversale externe, au moins une ouverture latérale de sortie étant aménagée dans l'aiguille à proximité de l'extrémité distale.

Selon l'invention, au moins une ouverture latérale de sortie est délimitée par un bord périphérique avec un profil arrondi de raccordement ne présentant pas d'arête saillante, entre les faces interne et externe de l'aiguille, et l'aiguille possède une paroi d'épaisseur qui, à partir de l'extrémité proximale et sur une longueur déterminée de l'aiguille, est supérieure à l'épaisseur du reste de l'aiguille qui est constante jusqu'à l'extrémité distale.

De plus, le vecteur d'injection selon l'invention peut comporter, en combinaison, au moins l'une et/ou l'autre des caractéristiques additionnelle suivantes :
- l'aiguille tubulaire possède, sur sensiblement un tiers de sa longueur partant de l'extrémité proximale, une paroi d'épaisseur qui est sensiblement double par rapport à l'épaisseur du reste de l'aiguille allant jusqu'à l'extrémité distale,
- l'aiguille tubulaire présente une zone de raccordement progressive entre les épaisseurs de la paroi de l'aiguille,
- l'ouverture latérale de sortie présente une surface telle que Si < So < 5 Si, avec Si égale à la surface de la section droite transversale interne de l'aiguille,
- l'ouverture latérale de sortie présente une profondeur radiale telle que Po ≤ De/1,7, avec De, le diamètre de la face externe de l'aiguille,
- l'ouverture latérale de sortie présente une longueur prise sensiblement parallèlement à l'axe longitudinal de l'aiguille, telle que De < Lo < 2,5 De, avec De, le diamètre de la face externe de l'aiguille,
- l'aiguille possède un diamètre externe compris entre 0,15 et 1 mm et de préférence entre 0,4 et 0,9 mm,
- l'aiguille possède une longueur comprise entre 2 et 15 cm et de préférence entre 4 et 10 cm.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue de face d'un exemple de réalisation d'un vecteur d'injection conforme à l'invention.
La **Figure 2** est une vue en coupe élévation prise sensiblement selon les lignes II-II de la **Fig. 1**.
La **Figure 3** est une coupe partielle prise sensiblement selon la ligne III de la **Fig. 2**.
La **Figure 4** est une vue en coupe d'un détail « A » du vecteur d'injection illustré à la **Fig. 2**.
La **Figure 5** est une vue de côté à plus grande échelle du détail « A » de l'extrémité distale du vecteur d'injection illustré à la **Fig. 2**.
La **Figure 6** est une vue en coupe transversale prise sensiblement selon les lignes VI-VI de la **Fig. 4**.
La **Figure 7** est une vue d'un autre exemple de réalisation d'un vecteur d'injection conforme à l'invention.
La **Figure 8** est une vue à plus grande échelle d'un détail « B » du vecteur d'injection illustré à la **Fig. 7**.

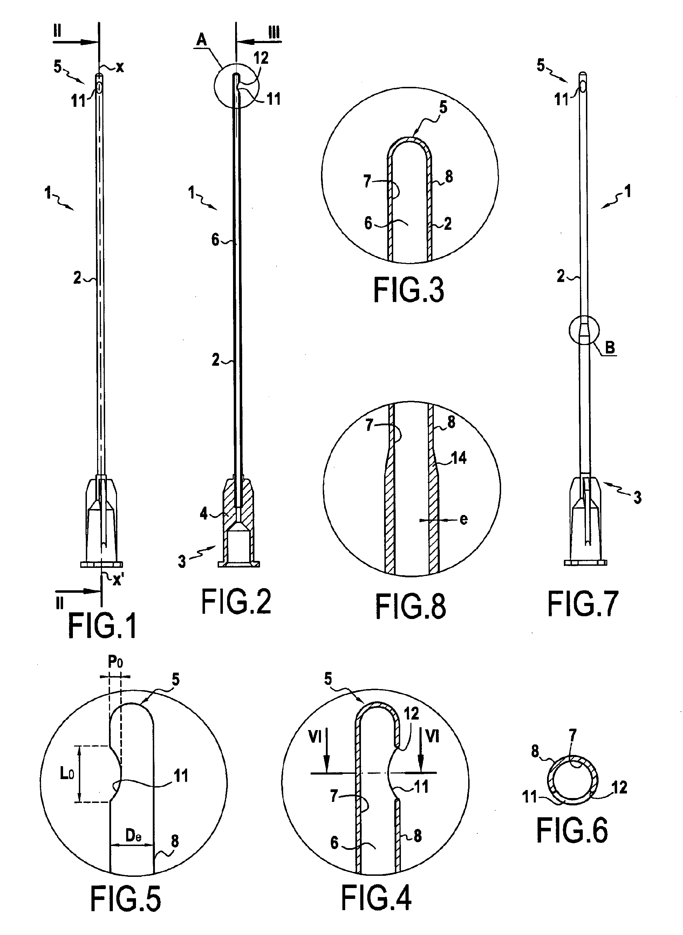

Tel que cela ressort plus précisément des **Fig. 1** et **2**, l'objet de l'invention concerne un vecteur d'injection **1** telle qu'une canule ou une aiguille au sens général adaptée pour l'injection (voire le prélèvement ?) d'un produit de remplissage au sens général dans le corps humain dans le domaine de la chirurgie esthétique telle que pour la micro-plastie ou la lipo-reconstruction. Par exemple, le vecteur d'injection **1** permet d'injecter pour la microplastie, de l'acide hyaluronique ou toute autre substance de remplissage, et pour la lipo-reconstruction, de la graisse autologue.

Ce vecteur d'injection **1** comporte une aiguille tubulaire **2** semi-rigide présentant un axe longitudinal de symétrie **xx**'. Avantageusement, cette aiguille tubulaire **2** est réalisée en un matériau métallique tel en acier inoxydable. Cette aiguille tubulaire **2** présente une extrémité proximale **3** de raccordement à un dispositif d'injection non représenté telle qu'une seringue. De manière classique, cette extrémité proximale **3** est pourvue d'un embout **4** d'adaptation du type par exemple « luer lock ».

Cette aiguille tubulaire **2** comporte à l'opposé de l'extrémité proximale **3,** une extrémité distale **5** arrondie ou hémisphérique fermée tel que cela ressort clairement de la **Fig. 3****.** L'aiguille tubulaire **2** présente ainsi une extrémité dite mousse atraumatique.

Cette aiguille tubulaire **2** présente un canal interne **6** délimité par une face interne tubulaire **7** présentant une section droite transversale interne **Si**. Cette aiguille tubulaire **2** présente une face externe tubulaire **8** délimitant une section droite transversale externe **Se**. De préférence, la section droite transversale interne **Si** de l'aiguille tubulaire **2** est circulaire. De même, la section droite transversale externe **Se** est circulaire.

L'aiguille tubulaire **2** présente ainsi une paroi d'épaisseur **e** constante sur une partie de sa longueur, à partir de l'extrémité distale **5** hémisphérique. L'aiguille tubulaire **2** est réalisée à partir d'une masse de métal unique de sorte qu'il n'apparaît aucune discontinuité ou joint entre l'extrémité distale hémisphérique **5** et la partie tubulaire de l'aiguille. Ainsi, la paroi de l'extrémité distale hémisphérique **5** et de l'aiguille tubulaire sur une partie de sa longueur présente une épaisseur **e** de valeur constante.

Tel que cela ressort plus précisément des **Fig. 4** à **6**, au moins une ouverture latérale de sortie **11** est aménagée dans l'aiguille tubulaire **2** à proximité de l'extrémité distale **5**. L'aiguille tubulaire **2** possède à partir de cette ouverture latérale de sortie **11** et en direction de l'extrémité distale **5,** une section droite transversale externe de valeur constante sur toute la longueur de la partie tubulaire de l'aiguille et qui diminue pour l'extrémité distale arrondie **5**.

Cette ouverture latérale de sortie **11** débouche ainsi sur les faces interne **7** et externe **8** de l'aiguille tubulaire **2** permettant ainsi une communication entre le canal interne **6** et l'extérieur de l'aiguille. De manière classique, cette ouverture latérale **11** assure la sortie du produit de remplissage provenant du canal interne **6** et introduit à l'aide d'un dispositif d'injection.

Cette ouverture latérale de sortie **11** est délimitée sur tout son pourtour par un bord périphérique **12** qui, comme cela apparaît clairement aux **Fig. 4** et **6**, présente un profil arrondi de raccordement entre les faces interne **7** et externe **8** de l'aiguille. En d'autres termes, le bord périphérique **12** ne présente pas d'arête saillante entre les faces interne **7** et externe **8** de l'aiguille. Le bord périphérique **12** présente ainsi un profil arrondi ou convexe selon toute la circonférence de l'ouverture latérale de sortie **11**. Il est à noter que l'extrémité distale **5** hémisphérique associée à l'ouverture latérale de sortie **11** sont parfaitement atraumatiques.

L'ouverture latérale de sortie **11** est ainsi parfaitement émoussée sur son périmètre en ne présentant aucune aspérité ou bavure de métal. Cet état de surface permet de réduire l'abrasion des tissus générant saignements et douleurs. Dans le même sens, la continuité de la surface de la face interne tubulaire **7** avec le bord **12** de l'ouverture latérale de sortie **11** permet un écoulement laminaire du produit de remplissage sans risque d'émulsionner le produit de remplissage au niveau de la rupture de profil liée à l'ouverture latérale de sortie **11**. De plus, cette continuité ou régularité de la surface de la face interne tubulaire **7** participe à la régulation automatique du débit du produit de remplissage, de par sa résistance hydrodynamique élevée et du fait du réglage de cette résistance par des proportions précises entre la section du canal interne **6** et la section de l'ouverture latérale de sortie **11** comme cela sera explicité dans la suite de la description.

Selon une caractéristique avantageuse de réalisation, l'ouverture latérale de sortie **11** présente une surface **So** telle que **Si** < **So** < 5 **Si** avec **Si** égale à la surface de la section droite transversale interne de l'aiguille. La surface **So** de l'ouverture latérale de sortie **11** correspond à la surface de l'orifice qui suit le profil ou la forme tubulaire de l'aiguille et plus précisément de la face **7**, **8**.

Selon une autre caractéristique avantageuse de réalisation, l'ouverture latérale de sortie **11** présente une profondeur radiale **Po** telle que **Po ≤ De**/1,7, avec **De**, le diamètre de la face tubulaire externe **8** de l'aiguille.

Selon une autre caractéristique avantageuse de réalisation, l'ouverture latérale de sortie **11** présente une longueur **Lo** prise selon l'axe longitudinale **x**, **x'** de l'aiguille telle que **De** < **Lo** < 2,5 **De**, avec **De**, le diamètre de la face tubulaire externe **8** de l'aiguille.

Il doit être considéré que le dimensionnement de l'aiguille tubulaire **2** par rapport à l'ouverture latérale de sortie **11** permet de réguler le débit du produit de remplissage afin d'obtenir un débit régulier et homogène en combinaison avec le profil arrondi du bord **12** de l'ouverture latérale de sortie **11**. L'aiguille tubulaire **2** possède ainsi des qualités hydrodynamiques maîtrisées permettant de réguler le début du produit de remplissage en fonction de sa viscosité. L'utilisation d'une aiguille tubulaire **2** selon l'invention évite l'injection irrégulière du produit de remplissage sous une forme de chapelet. La maîtrise de l'ensemble de ces caractéristiques permet de disposer d'une aiguille offrant une très grande efficacité pour chaque produit de remplissage et chaque geste spécialisé exécuté et notamment pour ceux les plus critiques concernant en particulier les traitements du regard, des cernes, du front et des pommettes.

De façon complémentaire, le vecteur d'injection **1** est miniaturisé permettant de passer en dessous du seuil des réactions douloureuses des terminaisons nerveuses cutanées. Il en résulte que les traitements mis en oeuvre à l'aide du vecteur d'injection **1** selon l'invention sont indolores.

Selon un exemple préféré d'application, l'aiguille possède un diamètre externe **De** compris entre 0,15 et 1 mm et de préférence entre 0,4 et 0,9 mm.

Selon un exemple de réalisation, l'aiguille possède une longueur comprise entre 2 et 15 cm et de préférence entre 4 et 10 cm.

Il est à noter que la paroi de l'aiguille possède une épaisseur **e** variable afin de s'adapter aux caractéristiques mécaniques et aux contraintes et besoins chirurgicaux. Les **Fig. 7** et **8** illustrent un exemple de réalisation dans lequel la paroi de l'aiguille tubulaire **2** possède une épaisseur variable. Selon cet exemple, la paroi de l'aiguille tubulaire **2** possède une épaisseur **e** variable par palier qui augmente en direction de l'extrémité proximale **3** de l'aiguille. Ainsi, la paroi de l'aiguille tubulaire **2** possède une épaisseur constante sur une première partie de l'aiguille tubulaire partant de l'extrémité distale **5** et une épaisseur constante supérieure à l'épaisseur de la première partie, sur une deuxième partie de l'aiguille tubulaire allant jusqu'à l'extrémité proximale **5** de l'aiguille tubulaire **2**. Par exemple, sur un tiers de la longueur de l'aiguille tubulaire **2** partant de l'extrémité proximale **3**, l'aiguille tubulaire **2** possède une épaisseur **e** qui est sensiblement double par rapport à l'épaisseur du reste de l'aiguille allant jusqu'à l'extrémité distale **5**. Tel que cela ressort plus précisément de la **Fig. 8**, l'aiguille tubulaire **2** présente une zone de raccordement progressive **14** entre les deux parties de l'aiguille tubulaire **2** d'épaisseurs différentes, cette zone de raccordement progressive **14** s'étendant sur une longueur limitée de l'aiguille tubulaire **2**. Il est à noter que de préférence, le diamètre de la face interne tubulaire **7** reste constant alors que le diamètre de la face externe tubulaire **8** varie.

L'aiguille tubulaire **2** possède ainsi des caractéristiques mécaniques telles que souplesse, flexibilité, résistance mécanique, adaptées aux contraintes et besoins chirurgicaux. Le gainage de rigidification de l'aiguille tubulaire **2** à partir de son extrémité proximale **3** permet de résister au clivage des tissus sans risque de rupture de l'aiguille tubulaire **2** compte tenu de sa petite taille.

L'invention n'est pas limitée aux exemples décrits et représentés car diverses modifications peuvent y être apportées sans sortir de son cadre.

## Revendications

1. **-** Vecteur d'injection d'un produit de remplissage notamment pour la microplastie ou la lipo-reconstruction, comportant une aiguille tubulaire (**2**) semi-rigide d'axe longitudinal présentant une extrémité distale (**5**) hémisphérique fermée et une extrémité proximale (**3**) de raccordement à un dispositif d'injection, cette aiguille présentant une face interne tubulaire (**7**) délimitant une section droite transversale interne (**Si**) et une face externe tubulaire (**8**) délimitant une section droite transversale externe (**Se**), au moins une ouverture latérale de sortie (**11**) étant aménagée dans l'aiguille à proximité de l'extrémité distale, **caractérisé en ce qu'**au moins une ouverture latérale de sortie (**11**) est délimitée par un bord périphérique (**12**) avec un profil arrondi de raccordement ne présentant pas d'arête saillante, entre les faces interne et externe de l'aiguille, et **en ce que** l'aiguille (**1**) possède une paroi d'épaisseur (**e**) qui, à partir de l'extrémité proximale (**3**) et sur une longueur déterminée de l'aiguille, est supérieure à l'épaisseur du reste de l'aiguille qui est constante jusqu'à l'extrémité distale (**5**).

2. - Vecteur d'injection selon la revendication 1, **caractérisé en ce que** l'aiguille tubulaire (**2**) possède, sur un tiers de sa longueur partant de l'extrémité proximale (**3**), une paroi d'épaisseur (**e**) qui est sensiblement double par rapport à l'épaisseur (**e**) du reste de l'aiguille allant jusqu'à l'extrémité distale (**5**).

3. - Vecteur d'injection selon la revendication 1 ou 2, **caractérisé en ce que** l'aiguille tubulaire (**2**) présente, une zone de raccordement progressive entre les deux parties de l'aiguille tubulaire d'épaisseur différente.

4. - Vecteur d'injection selon la revendication 1, **caractérisé en ce que** l'ouverture latérale de sortie (**11**) présente une surface (**So**) telle que **Si** < **So** < 5 **Si**, avec **Si** égale à la surface de la section droite transversale interne de l'aiguille.

5. - Vecteur d'injection selon la revendication 4, **caractérisé en ce que** l'ouverture latérale de sortie (**11**) présente une profondeur radiale (**Po**) telle que **Po** ≤ **De**/1,7, avec **De**, le diamètre de la face externe (**8**) de l'aiguille.

6. - Vecteur d'injection selon l'une des revendications 4 à 6, **caractérisé en ce que** l'ouverture latérale de sortie (**11**) présente une longueur (**Lo**) prise sensiblement parallèlement à l'axe longitudinal de l'aiguille, telle que **De** < **Lo** < 2,5 **De**, avec **De**, le diamètre de la face externe (**8**) de l'aiguille.

7. - Vecteur d'injection selon l'une des revendications 1 à 6, **caractérisé en ce que** l'aiguille (**2**) possède un diamètre externe (**De**) compris entre 0,15 et 1 mm et de préférence entre 0,4 et 0,9 mm.

8. - Vecteur d'injection selon l'une des revendications 1 à 7, **caractérisé en ce que** l'aiguille (**2**) possède une longueur comprise entre 2 et 15 cm et de préférence entre 4 et 10 cm.

## Patentansprüche

1. Träger zum Injizieren eines Füllproduktes insbesondere für die Mikroplastik oder die Liporeconstruction, umfassend eine röhrenförmige, halbstarre Nadel (2) mit einer Längsachse, die ein geschlossenes halbkugelförmiges distales Ende (5) und ein proximales Ende (3) zum Anschließen an eine Injektionsvorrichtung aufweist, wobei diese Nadel eine röhrenförmige Innenseite (7), die einen inneren Querschnitt (Si) begrenzt, und eine röhrenförmige Außenseite (8), die einen äußeren Querschnitt (Se) begrenzt, aufweist, wobei wenigstens eine seitliche Austrittsöffnung (11) in der Nadel in der Nähe des distalen Endes ausgebildet ist, **dadurch gekennzeichnet, dass** wenigstens eine seitliche Austrittsöffnung (11) durch einen Umfangsrand (12) mit einem abgerundeten Verbindungsprofil, der keine vorspringende Kante aufweist, zwischen der Innen- und der Außenseite der Nadel begrenzt ist, und dass die Nadel (2) eine Wand mit einer Dicke (e) besitzt, die ab dem proximalen Ende (3) und über eine bestimmte Länge der Nadel größer ist als die Dicke der restlichen Nadel, welche bis zu dem distalen Ende (5) konstant ist.

2. Injektionsträger nach Anspruch 1, **dadurch gekennzeichnet, dass** die röhrenförmige Nadel (2) über ein Drittel ihrer Länge, von dem proximalen Ende (3) ausgehend, eine Wand mit einer Dicke (e) besitzt, die im Vergleich zu der bis zu dem distalen Ende (5) gehenden Dicke (e) der restlichen Nadel im Wesentlichen das Doppelte beträgt.

3. Injektionsträger nach Anspruch 1 oder 2, **dadurch gekennzeichnet, dass** die röhrenförmige Nadel (2) einen schrittweisen Verbindungsbereich zwischen den beiden Teilen der röhrenförmigen Nadel mit unterschiedlicher Dicke aufweist.

4. Injektionsträger nach Anspruch 1, **dadurch gekennzeichnet, dass** die seitliche Austrittsöffnung (11) eine Fläche (So) aufweist, die derart ist, dass Si < So < 5 Si, wobei Si gleich der Fläche des inneren Querschnitts der Nadel ist.

5. Injektionsträger nach Anspruch 4, **dadurch gekennzeichnet, dass** die seitliche Austrittsöffnung (11) eine radiale Tiefe (Po) aufweist, die derart ist, dass Po ≤ De/1,7, wobei De der Durchmesser der Außenseite (8) der Nadel ist.

6. Injektionsträger nach einem der Ansprüche 4 bis 6, **dadurch gekennzeichnet, dass** die seitliche Austrittsöffnung (11) eine Länge (Lo), im Wesentlichen parallel zur Längsachse der Nadel gekommen, aufweist, die derart ist, dass De < Lo < 2,5 De, wobei De der Durchmesser der Außenseite (8) der Nadel ist.

7. Injektionsträger nach einem der Ansprüche 1 bis 6, **dadurch gekennzeichnet, dass** die Nadel (2) einen Außendurchmesser (De) im Bereich zwischen 0,15 und 1 mm und vorzugsweise zwischen 0,4 und 0,9 mm aufweist.

8. Injektionsträger nach einem der Ansprüche 1 bis 7, **dadurch gekennzeichnet, dass** die Nadel (2) eine Länge im Bereich zwischen 2 und 15 cm und vorzugsweise zwischen 4 und 10 cm aufweist.

## Claims

1. - A carrier for injecting a filler product, in particular for microplasty or lipo-reconstruction, comprising a semi-rigid tubular needle (**2**) having a longitudinal axis with a closed hemispherical distal end (**5**) and a proximal end (**3**) for connecting to an injection device, said needle having an inner tubular surface (**7**) defining an inner transverse cross-section (**Si**) and an outer tubular surface (**8**) defining an outer transverse cross-section (**Se**), wherein at least one output side opening (**11**) is formed in the needle in the vicinity of the distal end, **characterized in that** at least one output side opening (1**1**) is defined by a peripheral edge (**12**) with a rounded connection profile free of any protruding edge, between the inner and outer surfaces of the needle, and **in that** the wall of the needle (**2**) has a thickness (**e**), from the proximal end (**3**) and over a predetermined length of the needle, which is greater than the thickness of the needle which is constant up to the distal end (**5**).

2. - An injection carrier according to claim 1, **characterized in that** the tubular needle (**2**) has, over one third of its length starting from the proximal end (**3**), a wall with a thickness (**e**) that is substantially twice the thickness (**e**) of the rest of the needle up to the distal end (**5**).

3. - The injection carrier according to claim 1 or 2, **characterized in that** the tubular needle (**2**) has a gradual connecting zone between the two parts of the tubular needle of different thickness.

4. - The injection carrier according to claim 1, **characterized in that** the output side opening (**11**) has a surface (**So**) such that **Si** < **So** , 5 **Si**, with **Si** equal to the surface of the inner transverse cross-section of the needle.

5. - The injection carrier according to claim 4, **characterized in that** the output side opening (**11**) has a radial depth (**Po**) such that **Po** ≤ **De**/1.7, with **De** the diameter of the outer face (**8**) of the needle.

6. - The injection carrier according to one of claims 4 to 6, **characterized in that** the output side opening (**11**) has a length (**Lo**) taken substantially parallel to the longitudinal axis of the needle, such that **De** < **Lo** < 2.5 **De**, with **De** the diameter of the outer face (**8**) of the needle.

7. - The injection carrier according to one of claims 1 to 6, **characterized in that** the needle (**2**) has an outer diameter (**De**) between 0.15 and 1 mm and preferably between 0.4 and 0.9 mm.

8. - The injection carrier according to one of claims 1 to 7, **characterized in that** the needle (**2**) has a length between 2 and 15 cm and preferably between 4 and 10 cm.
